(19) Europäisches Patentamt / European Patent Office / Office européen des brevets

(11) **EP 4 691 438 A1**

(12) **EUROPEAN PATENT APPLICATION**
published in accordance with Art. 153(4) EPC

(43) Date of publication:
**11.02.2026 Bulletin 2026/07**

(21) Application number: **23930788.7**

(22) Date of filing: **25.10.2023**

(51) International Patent Classification (IPC):
***A61F 13/15*** (2006.01)

(52) Cooperative Patent Classification (CPC):
**A61F 13/15**

(86) International application number:
**PCT/JP2023/038503**

(87) International publication number:
**WO 2024/202158 (03.10.2024 Gazette 2024/40)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **30.03.2023 JP 2023055943**

(71) Applicant: **DAIO PAPER CORPORATION
Shikokuchuo-shi
Ehime 799-0492 (JP)**

(72) Inventors:
• **KURAMOCHI, Mihoko**
  **Sakura-shi, Tochigi 329-1411 (JP)**
• **TAKAHASHI, Hiromi**
  **Sakura-shi, Tochigi 329-1411 (JP)**

(74) Representative: **Epping - Hermann - Fischer
Patentanwaltsgesellschaft mbH
Schloßschmidstraße 5
80639 München (DE)**

(54) **INDIVIDUALLY-PACKAGED ABSORBENT ARTICLE**

(57)    In an individually packaged absorbent article formed by wrapping an absorbent article in a packaging sheet made of paper, a sealing portion is formed by folding the packaging sheet toward an inner surface side in a longitudinal direction of the packaging sheet and sealing both edge portions in a lateral direction orthogonal to the longitudinal direction, a heat sealing agent is provided on at least a region included in the sealing portion of one of the inner surface and the outer surface of the packaging sheet, and the packaging sheet is a barrier agent-uncoated sheet or a barrier agent-coated sheet, and a coating weight of the barrier agent is 10% or less with respect to a total basis weight of the packaging sheet after the heat sealing agent is provided.

EP 4 691 438 A1

**Description**

TECHNICAL FIELD

The present invention relates to individually packaged absorbent articles.

BACKGROUND ART

Many absorbent articles, such as sanitary napkins, panty liners, incontinence pads, and the like, are provided as individually packaged absorbent articles (individual packages) in a state of being individually packaged and sealed with a packaging sheet for reasons of hygiene during storage, convenience during carrying, and the like. As a packaging structure of the individually packaged absorbent article, for example, a form in which the absorbent article is arranged on the inner surface of the packaging sheet, the packaging sheet is folded to the inner surface side together with the absorbent article, and then both edge portions are sealed is widely known.

As the material of the packaging sheet, a resin film, a nonwoven fabric, or the like is still mainly used, but a packaging sheet made of paper is also known. In the case of a paper packaging sheet, the sheet material itself is not thermally fused, and therefore, a device for increasing the sealing strength of the sealing portions is required. For example, Patent Document 1 describes a technique of forming sealing portions by pressurizing both edge portions of a folded packaging sheet between a pair of rolls respectively having a first tooth row and a second tooth row that meshes with the first tooth row, on the respective surfaces.

RELATED ART DOCUMENTS

PATENT DOCUMENT

Patent Document 1: Japanese Unexamined Patent Publication No. 2022-24624

SUMMARY OF THE INVENTION

PROBLEMS TO BE SOLVED BY THE INVENTION

However, the sealing strength of the sealing portion formed with the technique described in Patent Document 1 cannot be made so large as compared with the sealing strength of a sealing portion formed by thermal fusion. Therefore, it is considered to form a sealing portion by using a heat sealing agent. In this case, in view of the fact that an addition of the heat sealing agent as an ingredient requires an additional cost, it is desired to efficiently use the heat sealing agent, that is, to more reliably form the sealing portion even with a relatively small amount of the heat sealing agent.

In view of the above, an object of one aspect of the present invention is to provide a technique that can more reliably form a sealing portion by heat sealing in an individually packaged absorbent article in which the absorbent article is wrapped in a paper packaging sheet.

MEANS FOR SOLVING THE PROBLEMS

One aspect of the present invention is an individually packaged absorbent article formed by wrapping an absorbent article in a packaging sheet made of paper, wherein a sealing portion is formed by folding the packaging sheet toward an inner surface side in a longitudinal direction of the packaging sheet and sealing both edge portions in a lateral direction orthogonal to the longitudinal direction, a heat sealing agent is provided on at least a region included in the sealing portion of one of the inner surface and the outer surface of the packaging sheet, and the packaging sheet is a barrier agent-uncoated sheet or a barrier agent-coated sheet, and a coating weight of the barrier agent is 10% or less with respect to a total basis weight of the packaging sheet after the heat sealing agent is provided.

EFFECTS OF THE INVENTION

According to one aspect of the present invention, a sealing portion can be more reliably formed through heat sealing in an individually packaged absorbent article in which an absorbent article is wrapped in a paper packaging sheet.

BRIEF DESCRIPTION OF THE DRAWINGS

[FIG. 1] FIG. 1 is a plan view of an individually packaged absorbent article according to an embodiment of the present invention.

[FIG. 2] FIG. 2 is a developed plan view of the individually packaged absorbent article of FIG. 1.

[FIG. 3] FIG. 3 is a sectional view taken along line I-I of FIG. 1.

[FIG. 4] FIG. 4 is a sectional view taken along line II-II of FIG. 1.

[FIG. 5] FIG. 5 is a diagram for explaining an effect of a heat sealing agent in the embodiment.

[FIG. 6] FIG. 6 is a diagram for explaining an effect of the heat sealing agent in the embodiment.

DESCRIPTION OF EMBODIMENTS

Hereinafter, embodiments of the present invention will be described with reference to the drawings. In the drawings, the same or corresponding components are denoted by the same reference numerals unless otherwise specified, and the description thereof may be omitted.

<Basic Structure of Individually Packaged Absorbent Article>

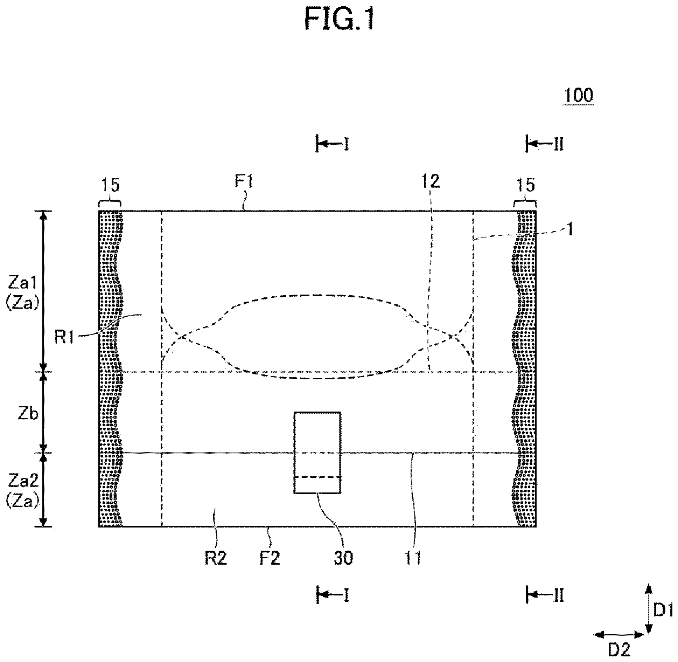

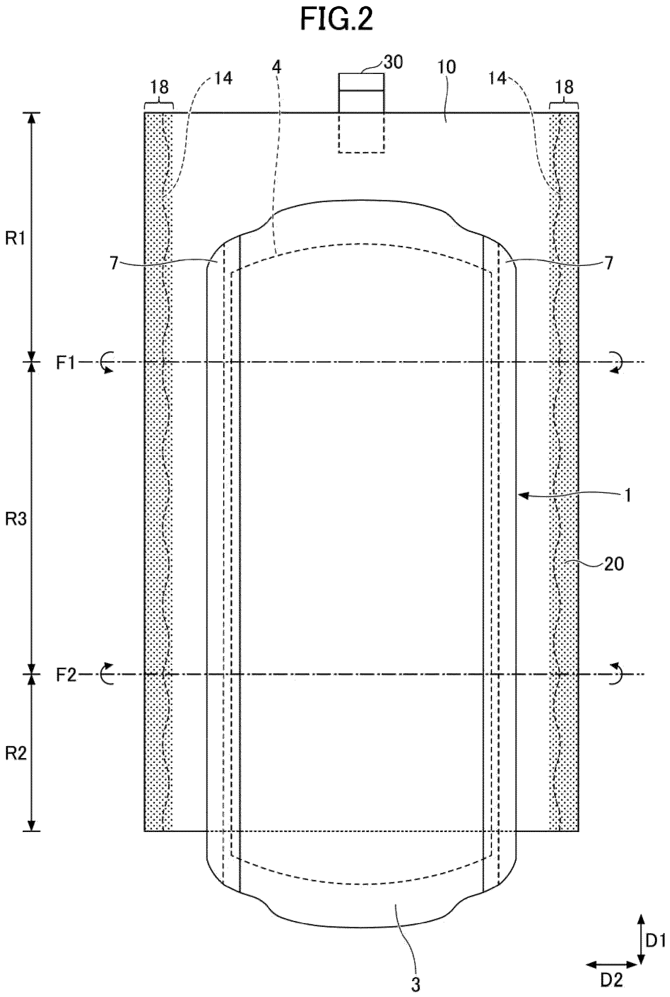

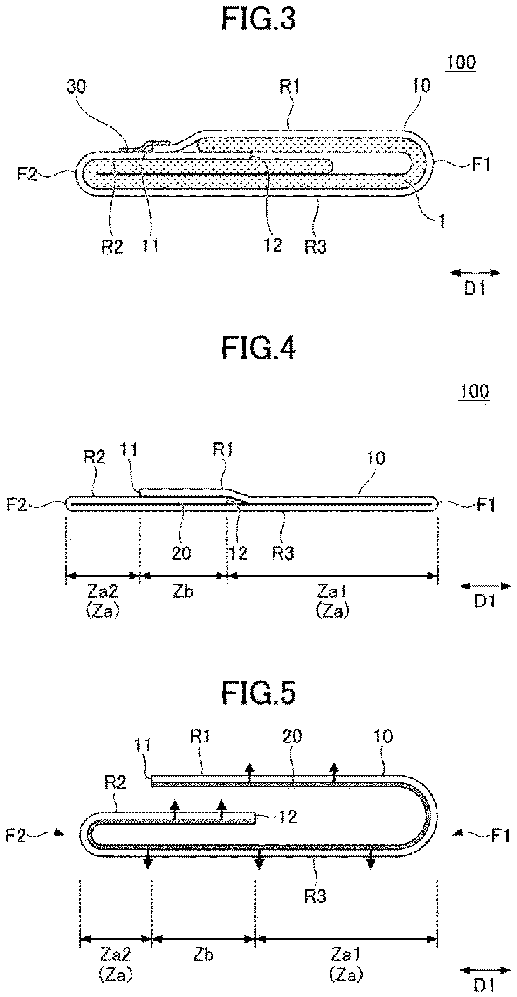

First, a basic structure of an individually packaged absorbent article will be described. FIG. 1 illustrates a plan view of an individually packaged absorbent article 100. FIG. 2 is a plan view of a packaging sheet 10 of the individually packaged absorbent article 100 of FIG. 1 in a developed state, as viewed from the inner surface of the packaging sheet 10, namely from the skin side of the absorbent article 1. FIG. 3 is a sectional view taken along line I-I of FIG. 1, and FIG. 4 is a sectional view taken along line II-II of FIG. 1.
As illustrated in FIGS. 1 through 4, the individually packaged absorbent article 100 includes a packaging sheet 10 and an absorbent article 1 individually packaged by the packaging sheet 10.

(Packaging Sheet)

As illustrated in FIG. 2, the packaging sheet 10 may have an elongated shape in a developed state, and has a longitudinal direction D1 and a lateral direction D2 orthogonal to the longitudinal direction. The longitudinal direction D1 and the lateral direction D2 of the packaging sheet 10 may correspond to the longitudinal direction and the lateral direction of the absorbent article 1, respectively. The shape of the packaging sheet 10 is not limited to a rectangular shape as illustrated in FIG. 2, and may be, for example, an oblong shape.
The dimensions of the packaging sheet 10 can be selected as appropriate according to the size and shape of the absorbent article 1 to be packaged. For example, the dimension of the longitudinal direction D1 (sometimes simply referred to as a "length") may be 100 mm to 450 mm, and the dimension of the lateral direction D2 (sometimes simply referred to as a "width") may be 70 mm to 250 mm, when the packaging sheet 10 is completely unfolded (not folded).
The packaging sheet 10 in the present embodiment is made of paper. The use of the paper packaging sheet 10 can contribute to reduction of plastics and achievement of sustainable development goals. Paper can also provide comfort to users because it can provide a unique texture, such as a gentle look and feel of a natural material. In this specification, "paper" refers to a thin flat sheet obtained by agglutinating plant fibers or other fibers with an agglutinating agent. In particular, "paper" refers to a sheet containing a plant fiber as a main raw material, for example, one containing 50% or more, preferably 80% or more, of a plant fiber, particularly a cellulose fiber, among the contained fibers. Examples of the plant fiber as a raw material of paper include wood pulp, non-wood pulp, waste paper pulp, and cotton cellulose, and the pulp may be any of mechanical pulp and chemical pulp. The plant fibers may be regenerated fibers such as rayon and cupra, or may be plant fibers recycled from a constituent element of an absorbent article and/or a packaging sheet for an absorbent article (recycled pulp or the like).
The paper may contain known paper additives, such as clay, calcium carbonate, starch, latex, color pigments, preservatives, and the like. Specific examples of the paper include various types of paper such as Western paper, Japanese paper, processed paper, and synthetic paper. Further, the paper may be paper conventionally used for other purposes, for example, paper called newsprint paper, printing paper (including high-quality paper), writing paper, drawing paper, packaging paper, tissue paper, hybrid paper, or the like. As thin paper, thin vellum paper, Indian paper, rice paper, glassine paper, tissue paper, toilet paper, filter paper, or the like may be used.
In the present specification, the paper packaging sheet mainly refers to a sheet containing the above-described paper. The paper packaging sheet may include a laminated sheet in which paper and a sheet made of a material other than paper are laminated, as well as a packaging sheet made of only paper. In the case where the packaging sheet 10 includes a sheet made of a material other than paper, the material other than paper may be a resin film, a nonwoven fabric, or the like. However, the packaging sheet 10 is particularly preferably made of paper without being laminated with a material sheet of a material other than paper, from the viewpoint of plastic reduction and/or from the viewpoint of being able to impart a natural texture unique to paper to a product.

A basis weight of the packaging sheet 10 used in the present embodiment may be preferably 8 $g/m^2$ to 50 $g/m^2$, more preferably 15 $g/m^2$ to 30 $g/m^2$ or less. By using the packaging sheet 10 having a basis weight in the above range, an individually packaged absorbent article having a good texture with a suppressed stiff feel can be obtained, and the generation of noise during carrying, unsealing, and the like can be prevented. The thickness of the packaging sheet 10 (the thickness after creping in the case of crepe paper) may be preferably 30 $\mu$m to 200 $\mu$m, and more preferably 35 $\mu$m to 100 $\mu$m. The basis weight of the packaging sheet is a basis weight of the packaging sheet in an unprocessed state, that is, a basis weight of the paper packaging sheet in a state where neither a barrier agent nor a heat sealing agent is applied.

The paper packaging sheet 10 in the present embodiment may be subjected to some processing. The processing may include, for example, processing for physically deforming the packaging sheet 10, such as creping, embossing, calendering, slitting, or plying, and may include processing for adding a substance to the packaging sheet 10, such as water repellent processing, mold release processing, or ink printing processing. However, when the processing of adding a substance is performed, the processing is preferably performed on a region other than the region where the heat sealing agent (described later in detail) is applied or a region other than the region corresponding to the sealing portion so as not to affect the sealing properties of the sealing portion. The ink printing is preferably applied to the outer surface of the packaging sheet, for example, and this improves the design of the individually packaged absorbent article 100. In the case where the ink printing is performed on the packaging sheet 10, it is preferable to use a material (uncreped paper or unembossed paper) that is not subjected to a process of forming irregularities on the surface of the packaging sheet, such as creping or embossing, as the packaging sheet 10.

(Absorbent Article)

The absorbent article 1 packaged by the packaging sheet 10 may be a flat and elongated article to be worn so as to face a discharge orifice of a body fluid (menstrual blood, vaginal discharge, urine, or the like). Specific examples of the absorbent article 1 may include a sanitary napkin, a panty liner, and a light incontinence pad. The description hereinafter is primarily based on an example where the absorbent article 1 is a sanitary napkin.

The absorbent article 1 may include, for example, as illustrated in FIG. 2, a liquid permeable top sheet 3, a liquid impermeable back sheet (not illustrated), and an absorber 4 arranged between the top sheet 3 and the back sheet.

As the back sheet, a sheet material having at least water impermeability, such as an olefin-based resin sheet of polyethylene, polypropylene, or the like, can be used. A laminated nonwoven fabric obtained by laminating a nonwoven fabric on a polyethylene sheet or the like, or a laminated sheet of a nonwoven fabric which is substantially liquid-impermeable by interposing a waterproof film therebetween may be used. Alternatively, a material having moisture permeability may be used.

As the top sheet 3, a porous or non-porous nonwoven fabric, a porous plastic sheet, or the like is preferably used. As the material fiber constituting the nonwoven fabric, for example, synthetic fibers, such as olefin such as polyethylene and polypropylene, polyester, and polyamide, regenerated fibers such as rayon and cupra, mixed fibers thereof, and natural fibers such as cotton, can be used alone or in combination of two or more kinds.

The absorber 4 is not limited as long as it is a material capable of absorbing and retaining body fluids, but preferably includes cotton-like pulp and a water-absorbent polymer. As the water-absorbent polymer, superabsorbent polymer granular powder (superabsorbent polymer (SAP)), superabsorbent polymer fiber (superabsorbent fiber (SAF)), and a combination thereof can be used. Examples of the pulp include chemical pulp obtained from wood, cellulose fibers such as dissolving pulp, and artificial cellulose fibers such as rayon and acetate. The pulp may be hardwood pulp obtained from hardwood, softwood pulp obtained from softwood, or a mixed pulp thereof. The pulp may be recycled pulp regenerated from used pulp.

The thickness of the absorber 4 may be 0.5 mm to 25 mm. The absorber 4 may have a structure in which a region facing the body fluid discharge orifice (a body fluid discharge orifice facing region) or a region facing the groove of the buttocks located behind the body fluid discharge orifice facing region is bulged. The absorber 4 has a size and shape that do not allow the absorber 4 to protrude from the top sheet 3 and the back sheet, and at the front and rear end edge portions of the absorber, the outer edges of the back sheet and the top sheet 3 are bonded by an adhesive such as hot melt or by an adhesion means such as heat sealing or ultrasonic sealing.

As illustrated in FIG. 2, in the periphery of the sides of the absorber 4 (the outer sides of the lateral direction D2), side sheets 7, 7 may be provided at respective ends of the lateral direction D2, along the longitudinal direction D1. As the side sheet 7, a water-repellent nonwoven fabric or a hydrophilic nonwoven fabric can be used.

In the example illustrated in FIGS. 1 through 4, the absorbent article 1 is a so-called wingless type, but may be configured as an article having wings extending to the sides. The wings may be formed by bonding the side sheets and the back sheet.

A displacement-prevention adhesive portion for preventing the absorbent article 1 from being displaced from underwear when the absorbent article 1 is attached to the underwear may be provided on the side of the back sheet of the absorbent article 1 (the non-skin side, namely the side facing the underwear when the absorbent article 1 is worn). The displacement-prevention adhesive portion may be formed in a plurality of bands extending in the longitudinal direction D1 or the lateral direction D2.

The total length of the absorbent article 1 can be 140 mm to 430 mm, and the breadth of the absorbent article 1 (the breadth of the main body excluding the wings in the case of having wings) can be 40 mm to 130 mm.

(Packaging Structure)

As illustrated in FIGS. 1 through 4, the absorbent article 1 is packaged by being folded together with the packaging sheet 10, and an individually packaged absorbent article 100 is thereby formed. When being folded, as illustrated in FIG. 2, the absorbent article 1 is placed on the inner surface of the packaging sheet 10 so that the back sheet side of the absorbent article 1 faces the inner surface of the packaging sheet 10. Then, the packaging sheet 10 and the absorbent article 1 are folded together toward the inner surface side in the longitudinal direction D1 along a first fold line F1 and a second fold line F2 along the width direction D2. More specifically, a first region R1 including one end 11 of the packaging sheet 10 in the longitudinal direction D1 is folded back in the longitudinal direction D1 along the first fold line F1, and a second region R2 including the other end 12 of the packaging sheet 10 in the longitudinal direction D1 is folded back along the second fold line F2. The region between the first region R1 and the second region R2 of the packaging sheet 10 is a third region R3. In the illustrated example, the packaging sheet 10 is folded in such a manner that the second region R2 is folded first and the first region R1 is folded to overlap the outer surface of the second region R2 (FIGS. 1 and 2), but the order of folding the first region R1 and the second region R2 may be reversed.

In the example illustrated in FIGS. 1 and 2, the packaging sheet 10 is inwardly folded into three sections together with the absorbent article 1 to wrap the absorbent article 1. Such a packaging folded into three sections can be formed relatively simply, and it is possible to package the absorbent article 1 hygienically and to easily take out the absorbent article 1, with such a packaging. The way of folding is not limited to folding into three sections, and may be folding into four or more sections (described later in detail), or may be folding into two sections.

In the three-ply configuration illustrated in FIGS. 1 through 4, the packaging sheet 10 is layered in three in the zone where the first region R1 and the second region R2 overlap, and the packaging sheet 10 is layered in two in the zone where the first region R1 and the second region R2 do not overlap. In FIGS. 1, 3 and 4, a zone where the packaging sheet 10 is layered in three is illustrated as a three-ply zone Zb, and zones where the packaging sheet 10 is layered in two is illustrated as two-ply zones Za1 and Za2 (Za altogether), respectively.

After the packaging sheet 10 is folded together with the absorbent article 1 to package the absorbent article 1, both edge portions of the lateral direction D2 are sealed along the longitudinal direction D1 to form the sealing portions 15, 15 (FIG. 1). As illustrated in FIG. 1, the sealing portions 15, 15 may be formed over the entire length of the longitudinal direction D1 of the packaging sheet 10. The sealing portions 15, 15 can prevent dust, dirt, a finger, or a small object from entering the individually packaged absorbent article 100 from the end edge of the lateral direction D2 (sealing properties can be obtained).

The sealing portion 15 may be formed within a range of 20 mm from the end edge of the lateral direction D2. The sealing portion 15 may be formed in the entire range or may be formed in a part of the range, for example, at a position away from the end edge of the lateral direction D2. A length (width) of the sealing portion 15 in the lateral direction D2 may be 3 mm to 15 mm. In the example illustrated in FIG. 1, the contour of the sealing portion 15 in the inner side of the lateral direction D2 is formed in a wavy line shape, but the contour of the sealing portion 15 of the inner side of the lateral direction D2 may be formed in a straight line having a constant length (width) of the lateral direction D2 over the longitudinal direction D1.

In the individually packaged absorbent article 100, a fastening tape 30 may be provided at the center of the width direction D2 so as to straddle the one end 11 of the packaging sheet 10, that is, so as to straddle the first region R1 and the second region R2 on the outer surface of the individually packaged absorbent article 100. The fastening tape 30 can prevent an object, a finger, or the like from entering and being caught between the first region R1 and the second region R2, and prevent the seal from being unintentionally unsealed before use. Further, since the first region R1 can be lifted up by holding the fastening tape 30 at the time of unsealing, an act of unsealing is facilitated.

<Heat Sealing Agent and Heat Sealing>

A heat sealing agent is used to form the sealing portions 15, 15 in the individually packaged absorbent article 100. The heat sealing agent may contain a thermoplastic resin, preferably a resin that softens or melts at 70 °C to 170 °C, and the heat sealing agent is preferably an olefin-based heat sealing agent. The heat sealing agent may be a water dispersion of resin particles. Specific examples of the heat sealing agent include "Chemipearl (registered trademark)" series manufactured by Mitsui Chemicals, Inc. Specific examples of the heat sealing agent include "Chemipearl (registered trademark)" series manufactured by Mitsui Chemicals, Inc.

By using the heat sealing agent, even when the packaging sheet 10 is made of paper, layers of the folded packaging sheet can be bonded to each other by sealing using heat (heat sealing). In the heat sealing, both edge portions of the packaging sheet 10 (both edge portions of the lateral direction D2 of the packaging sheet 10) after being folded as described above are inserted into, for example, a pair of rolls, and are pressurized and heated by both rolls, whereby the layers of the packaging sheet 10 can be pressed against each other in the thickness-wise direction. The pair of rolls may be, for example, a combination of a roll having a plurality of protrusions on the surface and a roll having no irregularities on the surface. In this case, the plurality of protrusions of one roll are pressed against the packaging sheet, and a plurality of pressed portions corresponding to the protrusions can be formed on the packaging sheet. In this pressed portion, if the heat sealing agent is interposed between the opposing layers of the packaging sheet, the heat sealing agent is melted or softened by heat and adheres to the packaging sheet, and then the heat sealing agent is cooled and solidified, whereby the packaging sheets and the heat sealing agent are strongly bonded to each other. Therefore, in the present embodiment using the heat sealing agent, the sealed portion can be formed more reliably (the sealing properties are high) as compared with a conventional sealing which utilizes physical deformation (formation of irregularities or the like) of the packaging sheet without using any heat sealing agent. Therefore, in this embodiment using the heat sealing agent, the sealed portion can be formed more reliably (the sealing property is high) as compared with the conventional sealing which utilizes physical deformation (formation of irregularities or the like) of the packaging sheet without using a heat sealing agent.

Furthermore, the sealing strength obtained by the heat sealing agent can be enhanced by impregnating the heat sealing agent into a packaging sheet made of paper. This is because the heat sealing agent softened or melted by heating and pressurizing by a heat sealing means enters between fibers of the paper, and the bonding between the paper packaging sheet and the heat sealing agent becomes strong. Furthermore, the softened or melted heat sealing agent is caused to seep out to the uncoated surface of the paper packaging sheet on the opposite side (back side) to the surface (coated surface) coated with the heat sealing agent, whereby the sealing strength can be further increased. In a state where the heat sealing agent is cooled and solidified after the heat sealing agent has seeped out to the opposite side of the paper packaging sheet, the solid heat sealing agent is continuously present from one side to the other side in the thickness direction of the paper packaging sheet, and therefore the bonding between the packaging sheet and the heat sealing agent is enhanced. In addition, by allowing the heat sealing agent to appear on the uncoated surface opposite to the coated surface on which the heat sealing agent is coated, the adhesiveness of the heat sealing agent can be exhibited even on the opposite side of the packaging sheet. Therefore, the sealing properties on both surfaces of the region of the packaging sheet arranged at the center in, for example, the zone of a packaging sheet folded into three sections (the second region R2 in the zone Zb, which is described later) can also be improved.

In general, many of paper sheets are provided with a barrier agent. A sheet coated with a barrier agent is called a "barrier agent-coated sheet". The barrier agent is applied for the purpose of preventing a predetermined agent (ink or the like) from seeping out (bleeding through) from the side on which the predetermined agent is applied to the opposite side in a step of applying the predetermined agent different from the barrier agent to the sheet surface. In many cases, the coating is continuously applied to the entire surface of the sheet. In the present specification, the barrier agent includes an agent applied for the purpose of antifouling, waterproofing (water repellency), mold release, and the like. Specific examples of the barrier agent include resins, such as silicone-based resins, paraffin-based resins, olefin-based resins (e.g., polyethylene (PE)), polyvinyl alcohol (PVA) resins, and fluororesins, and clay. The barrier agent can be applied to the inner or outer surface of a packaging sheet in the form of a solution or dispersion. Alternatively, a film-like resin molded body may be formed in advance and may be attached to the inner surface or the outer surface of a paper packaging sheet.

When the paper packaging sheet is coated with the above-described barrier agent in advance, the barrier agent may inhibit the above-described heat sealing agent from permeating into the paper packaging sheet. Therefore, from the viewpoint of promoting the permeation of the heat sealing agent to increase the sealing strength, it is preferable to suppress the coating amount of the barrier agent. More specifically, the coating weight of the barrier agent is 10% or less, preferably 5% or less, more preferably 1% or less, and still more preferably 0.5% or less, of the total basis weight of the packaging sheet. Herein, the total basis weight of the packaging sheet is the basis weight of the processed packaging sheet in the individually packaged absorbent article, which is a final product, that is, the basis weight after the packaging sheet is coated with, in the case where coating of the barrier agent is coated, the barrier agent and the heat sealing agent. Further, it is preferable that the coating weight of the barrier agent is 0%, in other words, the packaging sheet is a barrier agent-uncoated sheet which is not coated with the barrier agent.

As described above, in this embodiment, by using the barrier agent-uncoated sheet as the packaging sheet or by setting the coating weight of the barrier agent provided on the packaging sheet 10 to a predetermined amount or less even when the barrier agent-coated sheet is used, the permeation of the heat sealing agent into the paper packaging sheet is promoted during the heating and pressurization by the heat sealing means as described above. Therefore, the sealing strength of the sealing portion 15 can be increased as compared with the case where the same amount of the heat sealing agent is used but the heat sealing agent does not permeate into the packaging sheet 10.

Regardless of the presence or absence of the barrier agent, a paper packaging sheet having a low density may be used in order to promote the penetration of the heat sealing agent softened or melted by being heated and pressurized by the heat sealing means into the paper packaging sheet 10 and increase the sealing strength. In that case, the packing sheet 10 may have a density of 0.74 g/cm$^3$ or less, preferably 0.72 g/cm$^3$ or less, more preferably 0.71 g/cm$^3$ or less. In the case of the packaging sheet 10 having such a density, since the intervals between the fibers of the paper packaging sheet 10 are coarse, the heat sealing agent softened or melted by heating and pressurization can easily enter between the fibers, and the permeation of the heat sealing agent into the packaging sheet is promoted. In addition, the packaging sheet 10 may have a density of preferably 0.30 g/cm$^3$ or more, more preferably 0.40 g/cm$^3$ or more, and still more preferably 0.42 g/cm$^3$ or more, in order to ensure the strength of the individually packaged absorbent article 100 as the packaging sheet 10.

Furthermore, by setting the packaging sheet 10 to have a density of 0.42 g/cm$^3$ or more, preferably 0.55 g/cm$^3$ or more, and more preferably 0.60 g/cm$^3$ or more, the heat sealing agent can be made less likely to seep out to the opposite side of the packaging sheet 10 (bleeding-through can be prevented) in the step of applying the heat sealing agent, which is an initial stage of production, i.e., the step of applying the heat sealing agent in the form of an aqueous dispersion of polymer particles to the packaging sheet. When the heat sealing agent in the state of the aqueous dispersion of the resin particles bleed through during an application of the heat sealing agent, the heat sealing agent is highly likely to adhere to a member (e.g., the surface of a conveyor for transportation) of the manufacturing apparatus, which causes inconvenience. However, such inconvenience can be prevented by the above density.

The seepage of the heat sealing agent will be further described below with reference to the drawings. FIG. 5 is a schematic view of the packaging sheet 10 having the heat sealing agent 20 provided on the inner surface of the packaging sheet 10. FIG. 5 is a cross-sectional view of the packaging sheet 10 taken along line II-II of FIG. 1, in which the layers of the packaging sheet 10 are illustrated separated from each other. As illustrated in FIG. 5, the heat sealing agent 20 is in a state of adhering to the inner surface of the packaging sheet 10 immediately after application, but when heat and pressure are applied during heat sealing, the applied heat sealing agent 20, that is, the resin particles of the heat sealing agent, are softened or melted, and can enter the packaging sheet 10, specifically, between the fibers contained in the paper packaging sheet 10, and can seep out to the surface (outer surface) opposite to the surface to which the heat sealing agent 20 is applied.

In the case where the packaging sheet 10 is inwardly folded into three sections as illustrated in FIG. 5, the heat sealing agent 20 applied to the inner surface of the packaging sheet 10 in one portion where the first region R1 and the third region R3 face each other faces that in the other portion where the second region R2 and the third region R3 face each other; thus, a sufficient sealing strength can be obtained. On the other hand, at the portion where the first region R1 and the second region R2 face each other, the heat sealing agent 20 is applied only to one of the layers of the packaging sheet 10, and therefore, the sealing strength is slightly weak. For this reason, for example, in the case where the first region R1 is peeled off from the one end 11 at the time of unsealing, the three-ply zone Zb and the two-ply zone Za1 require different forces for peeling, which may cause a sense of discomfort at the time of unsealing or may cause the first region R1 in the three-ply zone Zb to be unintentionally peeled before unsealing. In contrast, in the present embodiment, the heat sealing agent 20 can seep out into the packaging sheet 10 and can further seep out to the outer surface by using a barrier agent-uncoated packaging sheet, or by using a barrier agent-coated packaging sheet having a coating weight of the barrier agent at a predetermined value or less with respect to the total basis weight of the packaging sheet after the heat sealing agent is applied, or by using a packaging sheet having a predetermined density. By allowing the heat sealing agent 20 to seep out through the inner surface to the outer surface of the second region R2, the heat sealing agent 20 in the first region R1 can face the heat sealing agent 20 in the second region R2 even at a portion where the first region R1 and the second region R2 face each other, and the sealing strength between the layers of the packaging sheet is increased. Thus, in the case where the first region R1 is peeled from the one end 11 at the time of unsealing, the difference in the forces required for peeling between the three-ply zone Zb and the two-ply zone Za1 is reduced, and the sense of discomfort can be suppressed, and the first region R1 in the three-ply zone Zb can be prevented from being unintentionally unsealed.

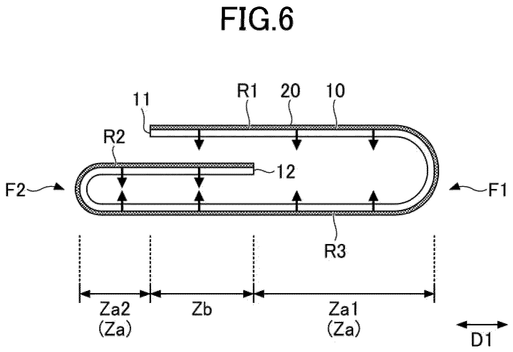

FIG. 6 is a schematic view of the packaging sheet 10 having the heat sealing agent 20 provided on the outer surface of the packaging sheet 10. FIG. 6 is also a cross-sectional view of the packaging sheet 10 taken along line II-II of FIG. 1, in which the layers of the packaging sheet 10 are illustrated separated from each other. In the case where the heat sealing agent 20 is applied to the outer surface of the packaging sheet 10, it is preferable to fold the packaging sheet 10 after the solvent (water) of the applied aqueous dispersion is volatilized and the heat sealing agent resin particles are fixed on the packaging sheet 10.

As illustrated in FIG. 6, the heat sealing agent 20 is in a state of adhering to the outer surface of the packaging sheet 10 immediately after application, but when heat and pressure are applied during heat sealing, the applied heat sealing agent 20, that is, the resin particles of the heat sealing agent, are softened or melted, and can enter the packaging sheet 10, specifically, between the fibers contained in the paper packaging sheet 10, and can seep out to the surface (inner surface) opposite to the surface to which the heat sealing agent 20 is applied.

In the example illustrated in FIG. 6, the heat sealing agent 20 is provided only on one side in the portion where the first region R1 and the second region R2 face each other, and the heat sealing agent 20 is not present between layers of the packaging sheet 10 in the other portions. Even in such a case, in the present embodiment, the heat sealing agent 20 can seep out into the packaging sheet 10 and can further seep out to the inner surface by using a barrier agent-uncoated packaging sheet, or by using a barrier agent-coated packaging sheet having a coating weight of the barrier agent of a predetermined value or less with respect to the total basis weight of the packaging sheet after the heat sealing agent is applied, or by using a packaging sheet having a predetermined density; sealing in the longitudinal direction D1 through heat sealing can be thereby achieved. Furthermore, the difference in sealing strength between the portion where the first region R1 and the second region R2 face each other and the portion where the first region R1 and the third region R3 face each other can be reduced. Thus, in the case where the first region R1 is peeled from the one end 11 at the time of unsealing, the difference in the forces required for peeling between the three-ply zone Zb and the two-ply zone Za1 is reduced, the sense of discomfort can be suppressed, and the first region R1 in the three-ply zone Zb can be prevented from being unintentionally unsealed.

In the present embodiment where the heat sealing agent seeps out, the difference between the sealing strength Saa of the bonding obtained by heat sealing the surfaces coated with the heat sealing agent (coated surfaces) facing each other (e.g., the bonding between the first region R1 and the third region R3 or the bonding between the second region R2 and the third region R3 in the example of FIG. 5) and the sealing strength San of the bonding obtained by heat-sealing the surface coated with the heat sealing agent (coated surface) and the surface not coated with the heat sealing agent (uncoated surface) facing each other (e.g., the bonding between the first region R1 and the second region R2 in the example of FIG. 5) can be reduced. Herein, the difference between Saa and San when Saa is 100%, that is, $(Saa - San) / Saa \times 100$, may be preferably 20% or less, more preferably 10% or less, still more preferably 5% or less, and still more preferably 3% or less.

The region of the packaging sheet 10 where the heat sealing agent is provided is not particularly limited, but may be a region included in at least the sealing portion 15 of the individually packaged absorbent article 100. The heat sealing agent may be provided on either the inner surface or the outer surface of the packaging sheet 10. FIG. 2 illustrates an example in which the heat sealing agent 20 is provided on the inner surface of the packaging sheet 10. In the example illustrated in FIG. 2, the heat sealing agent 20 is provided in a band shape in both edge regions 18, 18 of the lateral direction D2 of the packaging sheet 10, which include the regions 14, 14 where the sealing portions are to be formed. However, the heat sealing agent 20 may be provided in the regions 14, 14 where the sealing portions 15, 15 of the packaging sheet 10 are to be formed (sealing-portions-to-be-formed regions, whose outline is indicated by a dotted line in FIG. 2), and need not be provided in other regions. Thus, since the heat sealing agent 20 is applied to a minimum area to be pressed by heat sealing, the amount of the heat sealing agent 20 to be used can be reduced.

Examples of the means for applying the heat sealing agent 20 include flexographic printing, offset printing, screen printing, gravure printing, and application with a spray gun, a dispenser, or a roll coater. Among these, flexographic printing is preferable because a stable application amount and application pattern can be obtained, and an inexpensive aqueous ink can be used. The heat sealing agent may be applied in a solid manner or in a predetermined pattern, for example, a size-varying dots pattern.

<Examples>

(Experimental Example 1)

Paper packaging sheets of Examples 1-1 to 1-7 (100 mm in the MD (machine direction) $\times$ 100 mm in the CD (cross direction), not coated with a barrier agent) were prepared, and the basis weights, thicknesses, and the like were measured. A heat sealing agent was applied to each paper packaging sheet, and the bleed-through of the heat sealing agent during application and the sealing properties during sealing were evaluated. The packaging sheets used were as follows: Example 1-1: Unryu paper (manufactured by Maruishi Paper Tech Co., Ltd.), nominal basis weight 14 g/m$^2$ Example 1-2: Unryu paper (manufactured by Maruishi Paper Tech Co., Ltd.), nominal basis weight 17 g/m$^2$ Example 1-3: Unryu paper (manufactured by Maruishi Paper Tech Co., Ltd.), nominal basis weight 30 g/m$^2$ Example 1-4: FS rayon paper PM (manufactured by Maruishi Paper Tech Co., Ltd.), nominal basis weight 17 g/m$^2$

Example 1-5: FS rayon paper PM (manufactured by Maruishi Paper Tech Co., Ltd.), nominal basis weight 30 g/m$^2$
Example 1-6: Kinshachi paper (manufactured by Daio Paper Corporation), nominal basis weight 24.4 g/m$^2$
Example 1-7: Kinshachi paper (manufactured by Daio Paper Corporation), nominal basis weight 35 g/m$^2$

<Bleed-through Evaluation during Coating>

A heat sealing agent ("Chemipearl (registered trademark) S500" manufactured by Mitsui Chemicals, Inc.) was applied to the entire surface of one side of the paper packaging sheet of each example by a flexographic printer so as to form a solid coating having a basis weight of 1.8 g/m$^2$. The winding state of the packaging sheet around the center drum of the flexographic printing machine during the application was visually observed, and the bleed-through of the heat sealing agent during the application was evaluated. The evaluation criteria were as follows:

○: Bleed-through was not observed (operation was possible without winding)
△: Bleed-through was slightly observed (slight winding was observed and operability was poor)
✕: Distinct strike-through was observed (winding was observed and operation was impossible)

<Evaluation of Permeation after Heat Sealing>

The paper packaging sheet coated with the heat sealing agent as described above was inwardly folded into three sections as illustrated in FIG. 1 in the lengthwise direction on the side coated with the heat sealing agent, and the edge portion in the widthwise direction corresponding to the portion coated with the heat sealing agent was heat-sealed by inserting it between a pair of rolls to form a sealing portion having a width of 6 mm. One of the pair of rolls was a roll having protrusions on its surface for forming pressured portions, and the other was a flat roll having no irregularities on its surface, and the pair of rolls was arranged so that the axial direction thereof was along the longitudinal direction of the packaging sheet. The set roll pressure was 3.0 MPa, the roll temperature was 105 °C, and the heat sealing speed was 1.2 m/sec. The formed sealing portions were visually observed over the entire longitudinal direction of the packaging sheet, and the sealing properties were evaluated. The evaluation criteria were as follows:

○: The entire longitudinal direction was sealed
✕: Some portions were not sealed

The measured values and the evaluation results are shown in Table 1.

[Table 1]

| | Example 1-1 | Example 1-2 | Example 1-3 | Example 1-4 | Example 1-5 | Example 1-6 | Example 1-7 |
|---|---|---|---|---|---|---|---|
| Paper type | Unryu paper | | | FS rayon paper PM | | Kinshachi paper | |
| Basis weight (g/m$^2$) | 14.5 | 17.6 | 30.8 | 17.1 | 31.4 | 24.4 | 35.2 |
| Paper thickness ($\mu$m) | 35 | 41 | 51 | 34 | 55 | 35 | 47 |
| Density (g/cm$^3$) | 0.41 | 0.43 | 0.60 | 0.50 | 0.57 | 0.70 | 0.75 |
| Bleed-through at time of coating (operability) | ✕ | △ | N/A | △ | N/A | ○ | ○ |
| Permeation after heat sealing (sealing properties) | ○ | ○ | N/A | ○ | N/A | ○ | ✕ |

As shown in Table 1, it was found that high sealing properties were obtained in the packaging sheets (Example 1-1, Example 1-2, Example 1-4, and Example 1-6) having predetermined densities (0.74 g/cm$^3$ or less). Furthermore, it was also found that the bleed-through during the application of the heat sealing agent could be prevented in the packaging sheets (Example 1-2, Example 1-4, Example 1-6, and Example 1-7) having predetermined densities (0.42 g/cm$^3$ or more).

(Experimental Example 2)

A heat sealing agent ("Chemipearl (registered trademark)", 95 mm manufactured by Mitsui Chemicals, Inc.) was applied to the entire surface of one side of a paper packaging sheet (FS rayon paper PM, 95 mm in the MD × 35 mm in the CD, not coated with a barrier agent) by flexographic printing so as to have a basis weight of 2.7 g/m$^2$. In Example 2-1, two of the above packaging sheets were stacked, with the heat sealing agent-coated surfaces facing each other. In Example 2-2, two of the packaging sheets were stacked, with the coated surface and the uncoated surface facing each other. The sample of each example was sandwiched between a metal plate having protrusion portions for forming pressed portions on the surface and a flat metal plate having no irregularities on the surface, and was pressurized by an air type press machine and heat-sealed. The conditions of heat sealing were set to air of 0.8 MPa, press temperature of 115 °C, and heat sealing time of 100 seconds. The sealing strength of each example was measured and evaluated.

<Evaluation of Sealing Strength>

One of the packaging sheets was turned over by peeling off the end of the sealing portion of the sample of each example by 10 mm, and the respective end portions of the two packaging sheets were attached to chucks of a tensile tester (Tensilon tester "RTC1210" manufactured by Orientec Co., Ltd.), and the packaging sheets were pulled in opposite directions to measure a load (N). The measurement was performed at a chucking interval of 10 mm and a tensile speed of 100 mm/min. A maximum load in the measurement was defined as the sealing strength (peel strength) (N) of each example. In addition, in order to evaluate the difference between the sealing strength of Example 2-1 (bonding of the coated surfaces) and the sealing strength of Example 2-2 (bonding of the coated surface and the surface to be coated), the ratio of the difference in the sealing strength was obtained by the following formula:

Ratio of difference in sealing strength (%) = {(sealing strength of Example 2-1) - (sealing strength of Example 2-2)} / (sealing strength of Example 2-1) $\times$ 100

The results are shown in Table 2.

[Table 2]

| | Example 2-1 (Coated surface and coated surface) | Example 2-2 (Coated surface and uncoated surface) |
|---|---|---|
| Sealing strength (N) | 0.0886 | 0.0877 |
| **Ratio of difference** in sealing strength (%) *1 | - | 1.0 |
| *1:<br><br>Ratio of difference in sealing strength (%)<br><br>= {(Sealing strength of Example 2−1) − (Sealing strength of Example 2−2)}<br>/ (Sealing strength of Example 2−1) × 100 | | |

Table 2 shows that the difference between the bonding strength between the coated surfaces (Example 2-1) and the bonding strength between the coated surface and the uncoated surface (Example 2-2) was small. This is considered to be because, even when the heat sealing agent was applied only to one surface, the heat sealing agent sufficiently seeped out also to the surface of the packaging sheet opposite to the surface to which the heat sealing agent was applied, due to the heating and pressurization during the heat sealing.

The present invention has been described above based on the embodiments, but the present invention is not limited to these embodiments. The above-described embodiments can be variously changed, modified, replaced, added, deleted, combined, and the like within the scope described in the claims, and these also belong to the technical scope of the present invention. The above-described components can be discretionarily combined.

Preferred aspects of the present invention will be further described below.

A first aspect is an individually packaged absorbent article formed by wrapping an absorbent article in a packaging sheet made of paper, wherein a sealing portion is formed by folding the packaging sheet toward an inner surface side in a longitudinal direction of the packaging sheet and sealing both edge portions in a lateral direction orthogonal to the longitudinal direction, a heat sealing agent is provided on at least a region included in the sealing portion of one of the inner surface and the outer surface of the packaging sheet, and the packaging sheet is a barrier agent-uncoated sheet or a barrier agent-coated sheet, and a coating weight of the barrier agent is 10% or less with respect to a total basis weight of the packaging sheet after the heat sealing agent is provided.

According to the first aspect, the heat sealing agent is provided in at least the region included in the sealing portion on one of the inner surface and the outer surface of the packaging sheet. Therefore, even in the case of a paper packaging sheet, a heat sealing means can be used to form the sealing portions of both edge portions, and therefore, the packaging sheet can be sealed more reliably (sealing properties are high) as compared with the conventional sealing which does not use heat sealing, such as pressing of the packaging sheet between rows of teeth that mesh with each other.

In general, many of paper sheets are coated with a barrier agent. In other words, a barrier agent-coated sheet is often used. The barrier agent is applied in advance, for example, in a process of applying a predetermined agent (for example, ink, a functional agent other than ink, or the like) to the sheet surface, in order to prevent the predetermined agent from seeping out (bleed-through) from the side to which the agent is applied to the opposite side. However, in the case where the sealing portion of the individually packaged absorbent article is formed by heat sealing, more reliable sealing can be achieved by impregnating the paper packaging sheet with the above-mentioned heat sealing agent, and further by allowing the heat sealing agent to seep out to the opposite side of the packaging sheet. In the present aspect, as the packaging sheet, a sheet not coated with a barrier agent or a sheet coated with a barrier gent but having a reduced coating weight of the barrier agent is used. More specifically, the packing sheet is used in which the coating weight of the sealing agent is 10% or less of the total basis weight of the packing sheet after the heat sealing agent is applied. This makes it easy for the heat sealing agent, which is softened or melted during heating and pressurization by the heat sealing means, to penetrate between fibers of the paper packaging sheet, and the bonding between the packaging sheet and the heat sealing agent becomes stronger. Furthermore, in the case where the softened or melted heat sealing agent is seeped out to the side of the packaging sheet opposite to the side on which the heat sealing agent is applied, the heat sealing agent can be continuously present from one side to the other side in the thickness direction of the packaging sheet, and therefore, the bonding between the packaging sheet and the heat sealing agent is further strengthened. Since the heat sealing agent can be made to appear on the opposite side of the packaging sheet, the sealing strength on the opposite side can be improved. Therefore, according to the present aspect, even if the amount of the heat sealing agent is relatively small, it is possible to form a sealing portion having a higher sealing strength at both edge portions of the individually packaged absorbent article.

In a second aspect, the packaging sheet is a packaging sheet not coated with a barrier agent.

According to the second aspect, the heat sealing agent is more likely to permeate between fibers of the paper packaging sheet during heating and pressurization. Therefore, the above-described effect of improving the sealing strength of the sealing portion can be enhanced.

In a third aspect, the packaging sheet has a density of $0.30 \text{ g/cm}^3$ to $0.74 \text{ g/cm}^3$.

According to the third aspect, since the packaging sheet has a density in a predetermined relatively small range, the strength as the packaging sheet of the individually packaged absorbent article is secured, and the heat sealing agent is more likely to permeate between the fibers of the packaging sheet at the time of heating and pressurization, and thus, more reliable sealing can be performed, and the sealing strength of the sealing portion can be further improved.

In a fourth aspect, the packaging sheet has a basis weight of $15 \text{ g/cm}^3$ to $30 \text{ g/m}^2$.

According to the fourth aspect, by setting the basis weight (basis weight per unit area) of the packaging sheet to a predetermined range, the strength of the packaging sheet can be secured, and the stiff feeling when the individually packaged absorbent article is held in the hand can be reduced.

In a fifth aspect, the heat sealing agent is provided on the inner surface of the packaging sheet.

In a state where the packaging sheet is folded, a region where the inner surfaces of the packaging sheet face each other is large. According to the fifth aspect, since the heat sealing agent is provided on the inner surface of the packaging sheet, the layers of the packaging sheet are more reliably bonded to each other via the heat sealing agent. According to the present aspect, since the heat sealing agent is not present or is present only in a small amount on the outer surface at a stage before heat sealing (before thermal fusion), it is possible to reduce a possibility that the heat sealing agent adheres to a member of a manufacturing apparatus or the like.

In a sixth aspect, (Saa - San) / Saa $\times$ 100 $\leq$ 20 (%), wherein a sealing strength of a portion of the packaging sheet, where a coated surface coated with the heat sealing agent faces and is sealed together with another coated surface, is represented by Saa, and a sealing strength of a portion of the packaging sheet, where a coated surface coated with the heat sealing agent faces and is sealed together with an uncoated surface not coated with the heat sealing agent, is represented by San.

According to the sixth aspect, since the difference between the sealing strength of the bonding between the coated surfaces coated with the heat sealing agent and the sealing strength of the bonding between the coated surface coated with the heat sealing agent and the uncoated surface not coated with the heat sealing agent is small, even when the heat sealing agent is applied on the inner surface of the packaging sheet and the packaging sheet is folded into three sections, the force required for peeling can be made substantially uniform depending on the portion. Therefore, the entire packaging sheet can be peeled off without a sense of discomfort when the packaging sheet is unsealed.

A seventh aspect is an individually packaged absorbent article formed by wrapping an absorbent article in a packaging sheet made of paper, wherein a sealing portion is formed by folding the packaging sheet toward an inner surface side in a longitudinal direction of the packaging sheet and sealing both edge portions in a lateral direction orthogonal to the longitudinal direction, a heat sealing agent is provided on at least a region included in the sealing portion of one of the inner surface and the outer surface of the packaging sheet, and the packaging sheet has a density of 0.30 g/cm$^3$ to 0.74 g/cm$^3$.

According to the seventh aspect, the heat sealing agent is provided in at least the region included in the sealing portion on one of the inner surface and the outer surface of the packaging sheet. Therefore, since heat sealing can be used to form the sealing portions of both edge portions even in the case of a paper-made packaging sheet, the packaging sheet can be sealed more reliably (sealing properties are high) as compared with sealing without using heat sealing, for example by forming irregularities.

In the case where the sealing portion of the individually packaged absorbent article is formed by heat sealing, more reliable sealing can be achieved by impregnating the paper packaging sheet with the above-mentioned heat sealing agent, and further by allowing the heat sealing agent to seep out to the opposite side of the packaging sheet. In the present aspect, by setting the density of the paper packaging sheet within a predetermined range, the heat sealing agent easily permeates between fibers of the paper packaging sheet during heating and pressurization by a heat sealing means, and the bonding between the packaging sheet and the heat sealing agent becomes stronger. Furthermore, in the case where the heat sealing agent seeps out to the side of the packaging sheet opposite to the side on which the heat sealing agent is applied, the heat sealing agent can be continuously present from one side to the other side in the thickness direction of the packaging sheet, and therefore, the bonding between the packaging sheet and the heat sealing agent is further strengthened. Since the heat sealing agent can be made to appear on the opposite side of the packaging sheet, the sealing strength on the opposite side can be improved. Therefore, according to the present aspect, even if the amount of the heat sealing agent is relatively small, it is possible to form a sealing portion having a higher sealing strength at both edge portions of the individually packaged absorbent article.

This application claims priority based on Japanese Patent Application No. 2023-055943 filed on March 30, 2023, the entire contents of which are incorporated herein by reference.

REFERENCE SIGNS LIST

| | |
|---|---|
| 1 | Absorbent article |
| 3 | Top sheet |
| 4 | Absorber |
| 7 | Side sheet |
| 10 | Packaging sheet |
| 11 | One end in the longitudinal direction |
| 12 | Other end in the longitudinal direction |
| 14 | Sealing-portions-to-be-formed region |
| 15 | Sealing portion |
| 18 | Edge region |
| 20 | Heat sealing agent |
| 30 | Fastening tape |
| 100 | Individually packaged absorbent article |
| D1 | Longitudinal direction of the packaging sheet |

D2 Lateral direction of the packaging sheet
F1 First fold line
F2 Second fold line
R1 First region
R2 Second region
R3 Third region
Za, Za1, Za2 Two-ply zone
Zb Three-ply zone

**Claims**

1. An individually packaged absorbent article formed by wrapping an absorbent article in a packaging sheet made of paper, wherein

   a sealing portion is formed by folding the packaging sheet toward an inner surface side in a longitudinal direction of the packaging sheet and sealing both edge portions in a lateral direction orthogonal to the longitudinal direction, a heat sealing agent is provided on at least a region included in the sealing portion of one of the inner surface and the outer surface of the packaging sheet, and
   the packaging sheet is a barrier agent-uncoated sheet or a barrier agent-coated sheet, and a coating weight of the barrier agent is 10% or less with respect to a total basis weight of the packaging sheet after the heat sealing agent is provided.

2. The individually packaged absorbent article according to claim 1, wherein
   the packaging sheet is a packaging sheet not coated with a barrier agent.

3. The individually packaged absorbent article according to claim 1 or 2, wherein
   the packaging sheet has a density of 0.30 g/cm$^3$ to 0.74 g/cm$^3$.

4. The individually packaged absorbent article according to claim 1 or 2, wherein
   the packaging sheet has a basis weight of 15 g/m$^2$ to 30 g/m$^2$.

5. The individually packaged absorbent article according to claim 1 or 2, wherein
   the heat sealing agent is provided on the inner surface of the packaging sheet.

6. The individually packaged absorbent article according to claim 1 or 2, wherein
   (Saa - San) / Saa $\times$ 100 $\leq$ 20 (%), wherein a sealing strength of a portion of the packaging sheet, where a coated surface coated with the heat sealing agent faces and is sealed together with another coated surface, is represented by Saa, and a sealing strength of a portion of the packaging sheet, where a coated surface coated with the heat sealing agent faces and is sealed together with an uncoated surface not coated with the heat sealing agent, is represented by San.

7. An individually packaged absorbent article formed by wrapping an absorbent article in a packaging sheet made of paper, wherein

   a sealing portion is formed by folding the packaging sheet toward an inner surface side in a longitudinal direction of the packaging sheet and sealing both edge portions in a lateral direction orthogonal to the longitudinal direction, a heat sealing agent is provided on at least a region included in the sealing portion of one of the inner surface and the outer surface of the packaging sheet, and
   the packaging sheet has a density of 0.30 g/cm$^3$ to 0.74 g/cm$^3$.

# FIG.1

# FIG.2

# FIG.3

# FIG.4

# FIG.5

# FIG.6

## INTERNATIONAL SEARCH REPORT

| International application No. |
| --- |
| **PCT/JP2023/038503** |

| A. | CLASSIFICATION OF SUBJECT MATTER |
| --- | --- |

*A61F 13/15*(2006.01)i
FI:  A61F13/15 220

According to International Patent Classification (IPC) or to both national classification and IPC

| B. | FIELDS SEARCHED |
| --- | --- |

Minimum documentation searched (classification system followed by classification symbols)

A61F13/15

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| Y | JP 2005-270512 A (DAIO PAPER CORPORATION) 06 October 2005 (2005-10-06) paragraphs [0004]-[0029], fig. 1-6 | 1-5, 7 |
| A | | 6 |
| Y | JP 2021-153917 A (DAIO PAPER CORPORATION) 07 October 2021 (2021-10-07) paragraphs [0052]-[0056], [0103] | 1-5, 7 |
| A | | 6 |
| Y | JP 2023-18807 A (DAIO PAPER CORPORATION) 09 February 2023 (2023-02-09) paragraphs [0041]-[0050] | 3, 4, 7 |
| A | | 6 |
| A | JP 2023-32954 A (DAIO PAPER CORPORATION) 09 March 2023 (2023-03-09) paragraphs [0027]-[0094], fig. 1-16 | 1-7 |
| A | JP 9-48405 A (KAO CORPORATION) 18 February 1997 (1997-02-18) paragraphs [0015]-[0032], fig. 1-5 | 1-7 |

☐ Further documents are listed in the continuation of Box C.    ☑ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
| --- | --- |
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **29 November 2023** | **26 December 2023** |

| Name and mailing address of the ISA/JP | Authorized officer |
| --- | --- |
| **Japan Patent Office (ISA/JP)** <br> **3-4-3 Kasumigaseki, Chiyoda-ku, Tokyo 100-8915** <br> **Japan** | |
| | Telephone No. |

Form PCT/ISA/210 (second sheet) (January 2015)

**INTERNATIONAL SEARCH REPORT**
Information on patent family members

International application No.

**PCT/JP2023/038503**

| Patent document cited in search report | | | Publication date (day/month/year) | Patent family member(s) | Publication date (day/month/year) |
|---|---|---|---|---|---|
| JP | 2005-270512 | A | 06 October 2005 | (Family: none) | |
| JP | 2021-153917 | A | 07 October 2021 | (Family: none) | |
| JP | 2023-18807 | A | 09 February 2023 | (Family: none) | |
| JP | 2023-32954 | A | 09 March 2023 | (Family: none) | |
| JP | 9-48405 | A | 18 February 1997 | (Family: none) | |

Form PCT/ISA/210 (patent family annex) (January 2015)

**REFERENCES CITED IN THE DESCRIPTION**

**Patent documents cited in the description**

- JP 2022024624 A
- JP 2023055943 A